# NEUE EUROPÄISCHE PATENTSCHRIFT

(11) **EP 0 111 249 B2**
(45) Veröffentlichungstag und Bekanntmachung des Hinweises auf die Entscheidung über den Einspruch: **15.02.1995**
(45) Hinweis auf die Patenterteilung: 31.05.1989
(21) Anmeldenummer: 83112026.6
(22) Anmeldetag: 30.11.1983
(51) Int. Cl.: A61L 2/24, A61L 2/18, A61C 3/00, A61B 19/00, A61C 19/00

(54) **Verfahren und Einrichtung zur Desinfektion von Wasserwegen in zahnmedizinischen Geräten.**
Method and device for disinfecting water ducts in dental appliances.
Procédé et installation pour la désinfection des conduits d'eau dans des appareils dentaires.

(30) Priorität: 14.12.1982 DE 3246266
(43) Veröffentlichungstag der Anmeldung: 20.06.1984
(73) Patentinhaber: SIEMENS AKTIENGESELLSCHAFT, 80333 München (DE)
(72) Erfinder: Ciszewski, Hans-Joachim, D-6141 Einhausen (DE); Pabst, Josef, D-6805 Heddesheim (DE); Weisser, Hans-Jörg, D-6140 Bensheim (DE)

(56) Entgegenhaltungen:
- DE-A- 2 836 532
- DE-A- 2 915 806
- DE-A- 2 938 400
- DE-A- 3 028 550
- DE-A- 3 032 475
- Acta path. Microbiol. Scand. Sect. B, 86, 1978, Seiten 101-106
- Minerva Nefrologica, Lavori originals , 26, 1979 Seiten 571-578
- Artificial Organs, Vol. 5, No. 3, August 1981, Seiten 269-277
- Dialysis & Transplantation, Bd. 11, No. 8, August 1982, Seiten 690-692
- Trans. Am. Soc. Artif. Intern. Organs, Bd. XXVIII, 1982, Seiten 287-290

## Beschreibung

Zahnmedizinische, Geräte weisen, ausgehend von der Frischwasserzufuhr am Eingang des Gerätes bis zu den einzelnen Austrittsstellen an den Verbrauchern, eine Vielzahl von häufig recht verzweigten Wasserwegen auf. Ein Problem stellt dabei die Beseitigung von Keimen und Bakterien bzw. Verhinderung einer Neubildung solcher Mikroorganismen in diesem Wasserwegenetz dar, insbesondere deswegen, weil das Wachstum dieser Mikroorganismen durch verschiedene Umstände, wie z. B. relativ geringe Entnahmemenge, wechselnde, teilweise relativ hohe Standzeiten, dem Wachstum förderliche Temperaturbereiche, dort besonders begünstigt ist.

Aus der DE-A-3 028 550 ist es bekannt, zur Entkeimung von Wasser in zahnmedizinischen Geräten Entkeimungsflüssigkeit mittels einer in Abhängigkeit von der Wasserentnahme gesteuerten Dosierpumpe periodisch in die zu den Verbrauchern führenden Leitungen einzugeben. Bei stark kontaminierten Wasserwegen ist wegen der Problematik, die in der Anwendung hoher Desinfektionsmittelkonzentrationen liegt, eine Reduzierung der Keimzahlen auf Werte, die für eine Trinkwasserqualität gefordert werden, nur nach längerer Anwendungszeit oder gar nicht möglich.

Die DE-A-2 938 400 beschreibt eine Einrichtung zur Desinfektion von Schläuchen, Rohrleitungen od.dgl., die in Spül- und Saugpumpen von medizinischen Apparaten und Geräten eingesetzt werden. Bei dieser bekannten Einrichtung wird mit Hilfe einer Druckquelle, z. B. mittels einer Luftpumpe, aus einem Behälter flüssiges Desinfektionsmittel durch die zu desinfizierenden Hohlräume der Gegenstände gefördert. Das Fördern erfolgt takt- bzw. impulsweise, indem in den Leitungsweg ein Schaltventil eingebaut ist, welches mit dem unter Druck gehaltenen Desinfektionsbehälter und mit dem zu desinfizierenden Schlauch (bzw. Rohr) in Verbindung steht, wobei das Desinfektionsmittel nach Spülung des Schlauch- oder Rohrleitungshohlraumes in einem weiteren Behälter aufgefangen wird. Nach der Desinfektion wird durch den betreffenden Hohlraum steriles Gas oder steriles Wasser eingeleitet, um evtl. noch vorhandene Reste an Desinfektionsmittel aus dem Hohlraum zu entfernen. Das Schaltventil wird von einer Programmsteuerung aus gesteuert, wobei mittels der Druckquelle (Luftpumpe), die den Desinfektionsmittelbehälter ständig unter Druck hält, bei geöffnetem Ventil das Desinfektionsmittel in die Hohlkammer gefördert wird. Im Gegensatz zu der vorgenannten Druckschrift wird in diesem Dokument kein Verfahren zur Desinfektion der Wasserwege in einem zahnärztlichen Gerät beschrieben. Des weiteren enthält die in diesem Dokument beschriebene Einrichtung keine Wasserverbraucher, wie Turbinenhandstücke, Spritzhandstücke od.dgl.

Der in den Ansprüchen 1 und 13 angegebenen Erfindung liegt die Aufgabe zugrunde, ein Verfahren und eine Einrichtung anzugeben bei welchem(r) man über gewisse Zeiträume ganz auf die Anwendung von Desinfektionsmitteln verzichten kann.

Notwendige Kontrollen werden zur Entlastung des Praxispersonals von z. B. einem Mikroprozessor oder vergleichbaren Steuereinrichtungen übernommen.

Vorteilhafte Ausgestaltungen und Weiterbildungen des erfindungsgemäßen Verfahrens und der erfindungsgemäßen Einrichtung sind in den Unteransprüchen enthalten.

Eine besonders wirksame Sanierung der Wasserwege erhält man bei Verwendung von Chlorhexidindigluconat, und zwar in einer Konzentration zwischen 0,05 und 0,2 % im Wasser. Um eine Neuverkeimung nach einer Gesamtdesinfektion nach dem genannten Verfahren wirksam zu verhindern, kann mittels einer Dosiereinrichtung eine Menge zwischen 5 ppm und 1 ppm des genannten Desinfektionsmittels dem Wasser zudosiert werden. Alternativ hierzu wird vorgeschlagen, nach der Desinfektion mit Chlorhexidindigluconat ein Silberpräparat in einer Menge von 0,05 bis 0,1 mg/l Wasser zuzudosieren.

Anhand der Zeichnung werden das erfindungsgemäße Verfahren sowie eine nach diesem Verfahren arbeitende Einrichtung näher beschrieben. Es zeigen:
Figur 1 einen stark vereinfachten Hydraulikplan mit Darstellung der Wasserwege und Verbraucher bei einem zahnärztlichen Gerät,
Figur 2 einen vereinfachten Übersichtsplan für die gesamte Ablaufsteuerung zur Durchführung des Verfahrens.

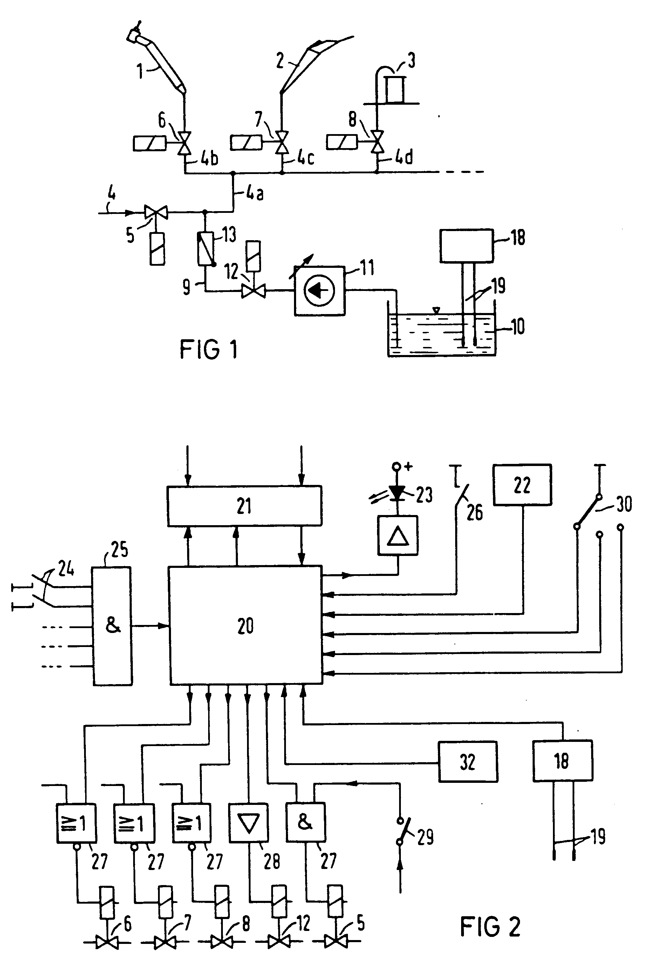

Im Ausführungsbeispiel nach Figur 1 wird von einem in der Zeichnung nicht dargestellten zahnärztlichen Gerät ausgegangen, welches Verbraucher 1 bis 3 enthält, namentlich ein Turbinenhandstück 1, ein Spritzhandstück 2 und eine Mundglasfülleinrichtung 3. Den Verbrauchern 1 bis 3 wird über eine Zufuhrleitung 4 aus dem Wasserleitungsnetz Frischwasser zugeführt. Ein erstes Magnetventil 5 am Eingang des Gerätes, z. B. am Geräteanschlußkasten, sperrt die Zufuhr des Frischwassers zentral ab. In den einzelnen, zu den Verbrauchern 1 bis 3 führenden Wasserwegen, deren Leitungsabschnitte mit 4a bis 4d bezeichnet sind, sind weitere Absperrmittel 6 bis 8 angeordnet. Diese Absperrmittel können Magnetventile oder auch pneumatisch gesteuerte Ventile sein, die entweder im Gerät oder in den Verbrauchern selbst, wie z. B. bei Spritzhandstücken üblich, angeordnet sein können.

In den nach dem zentralen Absperrventil 5 liegenden Leitungsabschnitt 4a mündet eine Dosierleitung 9 ein, über die von einem Behälter 10 mittels einer Dosiereinrichtung, z. B. einer Pumpe 11, Desinfektionsmittel eingebracht werden können. In der Dosierleitung 9 sind ferner ein Absperrventil 12 sowie ein Rückschlagventil 13 angeordnet. Der Behälter 10 enthält Desinfektionsmittel in einer Konzentration, die für eine Langzeitdesinfektion ausreicht und damit eine "Sanierung" der Leitungswege für längere Zeit, z. B. 2 bis 4 Wochen, sicherstellt.

Die Ansteuerung der Dosereinrichtung ermöglicht den Betriebszustand Dauerförderung des Desinfektionsmittels oder den Betrieb der Dosiereinrichtung in einem fest vorgegebenen oder variablen Betriebs-, Pausen-Verhältnis, so daß die Dosiereinrichtung kontinuierlich oder getaktet das Desinfektionsmittel fördern kann.

Damit ist es möglich, das Desinfektionsmittel, wie nachfolgend noch näher beschrieben, direkt in die Wasserwege zu pumpen, bis diese damit vollständig gefüllt sind und im Anschluß daran z. B. bei einer Konzentration des Desinfektionsmittels von 0,05 % mit dem Verhältnis 1 : 100 Wasserverbrauch zu Förderleistung der Dosiereinrichtung 5 PPM Desinfektionsmittel dem Wasser beizumischen.

Zur Kontrolle des Desinfektionsmittels oder, wenn die Anwendung unterschiedlicher Konzentrationen des Desinfektionsmittels für "Sanierung" und "Dauerdosierung" erforderlich ist, die durch das oben beschriebene Verfahren der Anpassung der Förderleistung der Dosiereinrichtung an den Wasserverbrauch nicht zu erreichen ist, kann ein in Figur 1 gezeigter Konzentrationsmelder 18 eine fehlerhafte Konzentration des Desinfektionsmittels erkennen und melden oder die Betriebsart "Sanieren" bzw. "Dauerdosierung" vorgeben.

Nachfolgend wird der Verfahrensablauf für eine Langzeitdesinfektion, d.h. für eine "Sanierung" der Wasserwege, bei bereits gegebener Verkeimung geschildert.

Zunächst wird das Magnetventil 5 geschlossen. so daß die Wasserzufuhr aus dem Leitungsnetz unterbrochen wird. Danach werden die den einzelnen Verbrauchern zugeordneten Absperrmittel, im Ausführungsbeispiel also die Ventile 6 bis 8, geöffnet. Sodann wird die Dosiereinrichtung 11 eingeschaltet, die nach Öffnen des in der Leitung 9 angeordneten Magnetventils 12 aus dem Behälter 10 Desinfektionsmittel in die Wasserwege 4a bis 4d einbringt. Dieser Vorgang wird zweckmäßigerweise über ein Zeitglied gesteuert, welches so ausgelegt ist, daß das Desinfektionsmittel so lange zugeführt wird, bis dieses an den jeweiligen Austrittsstellen austritt, also das gesamte, vorher in den Wasserwegen enthaltene Wasser ausgespült ist. Zweckmäßigerweise wird man bei diesem Füllvorgang die beiden Handstücke 1 und 2 in ein Auffanggefäß legen. Je nach Volumen des Wasserwegenetzes und Förderleistung der Dosierpumpe ergeben sich unterschiedliche Füllzeiten. Nach dem Füllen werden die Absperrmittel der Verbraucher geschlossen. Das Desinfektionsmittel bleibt nun in dem Wasserwegenetz eine bestimmte Zeit.

Nach Ablauf dieser Einwirkzeit, die bis zu 24 Stunden betragen kann, wird die Frischwasserzufuhr durch Öffnen des Magnetventils 5 wieder eingeschaltet und dabei das Desinfektionsmittel aus den Wasserwegen verdrängt. Das in die Leitung eingeschaltete Rückschlagventil 13 verhindert, daß Frischwasser in den Behälter 10 zurückfließen kann.

Als Desinfektionsmittel hat sich Chlorhexidindigluconat als besonders vorteilhaft erwiesen, und zwar für eine Langzeitdesinfektion in einer Konzentration zwischen 0,05 und 0,2 Der beschriebene Sanierungsprozeß kann automatisch alle 2 bis 4 Wochen durchgeführt werden. In der Zwischenzeit kann in der Regel auf die Anwendung von Desinfektionsmittel verzichtet werden. Denkbar wäre es aber auch, nach einer Sanierung der Wasserwege für eine ständige Freihaltung der Wasserwege von Keimen und Bakterien dadurch zu sorgen, daß dem Wasser mittels der Dosiereinrichtung 11 eine Menge zwischen 5 ppm und 1 ppm an Chlorhexidindigluconat zudosiert wird. Damit läßt sich eine Neuverkeimung wirksam verhindern. Anstelle von Chlorhexidin kann auch ein Silberpräparat verwendet werden. Die Zudosierungsmenge liegt hier zwischen 0,05 und 0,1 mg/l Wasser.

Die gesamte Steuerung des Verfahrensablaufes kann, wie anhand der Figur 2 näher erläutert, zweckmäßigerweise über ein mikroprozessorgesteuertes Zeitglied erfolgen, welches durch Tastendruck ausgelöst wird.

Anhand der Figur 2 wird der Ablauf des erfindungsgemäßen Verfahrens beschrieben.

Zentrales Glied der Ablaufsteuereinrichtung ist ein Mikroprozessor 20, der die eingehenden Signale, wie nachfolgend noch näher erläutert, verarbeitet und Signale zum schalten der diversen Magnetventile bzw. Anzeigesignale liefert. Die Programmierung der Füllzeiten, der Einwirkzeit, der Spülzeit und der Zeit, zu der eine erneute "Sanierung" erforderlich ist (in der Regel nach ca. 2 bis 4 Wochen), erfolgt in einem Zeitglied 21 (Timer) bzw. 22 (Behandlungspausenzeitspeicher), weiche zum besseren Verständnis separat dargestellt, jedoch auch im Mikroprozessor 20 enthalten sein können. Eine Leuchtdiode 23 zeigt zunächst durch Aufleuchten bzw. Blinken an, wann die Pausenzeit beendet und eine Desinfektion des Gerätes nötig ist.

Der Benutzer hat nun, wie zuvor bereits beschrieben, die Handstücke 1 und 2, die in der Regel in entsprechenden Ablagen abgelegt sind, aus diesen zu entnehmen und, um die während des Desinfiziervorganges anstehende Flüssigkeit aufzufangen, zweckmäßigerweise in ein Gefäß zu legen. Bei Entnahme der Handstücke aus den Ablagen werden über Signalgeber 24, die für jedes Handstück vorhanden sind, Signale an eine UND-Gatter-Verknüpfung 25 gegeben. Nur wenn alle Handstücke entnommen sind, erhält die Ablaufsteuereinrichtung 20 ein H-Signal und ist damit zur Ansteuerung der bereits geschilderten Verfahrensschritte vorbereitet. Wird nun eine Desinfektionstaste 26 gedrückt, so wird damit der Verfahrensablauf in Gang gesetzt, d.h. zunächst wird die Spule des Magnetventils 5 im Sinne einer Absperrung des Ventils geschaltet. Anschließend werden die Spulen der Ventile 6 bis 8 im Sinne einer Öffnung der Ventile geschaltet. Danach wird die Spule des Ventils 12 erregt und gleichzeitig die Pumpe der Dosiereinrichtung 11 eingeschaltet. Desinfektionsmittel wird nun aus dem Behälter 10 in die einzelnen Leitungsabschnitte 4a bis 4d gepumpt. Um die verschiedenen Leitungswiderstände zu berücksichtigen, werden die Magnetventile 6 bis 8 vorteilhafterweise nacheinander geöffnet. Diese Steuerung erfolgt ebenfalls über den Mikroprozessor 20. Die Ansteuerung der Magnetventilspulen erfolgt über Logikglieder 27 und Verstärker 28. Zuvor muß natürlich der Hauptschalter 29 eingeschaltet sein.

Nach dem Desinfizieren wird das Wasserhauptventil 5 wieder geöffnet und die Desinfektionsflüssigkeit aus dem Leitungssystem ausgetrieben.

Mit 30 ist ein Desinfektionsartenwahlschalter bezeichnet, mit dem von Langzeitdesinfektion auf Dauerdosierung oder Dauerdosierung mit Langzeitdesinfektionsmeldung umgeschaltet werden kann. Bei letzterer wird über die Anzeigelampe 23 angezeigt, wann eine erneute Sanierung des Gerätes notwendig ist.

Eine weitere Möglichkeit des Steuersystems 20, 21, 22 kann darin liegen, daß eine längere Betriebspaus, z. B. bei Urlaub, dadurch erkannt wird, daß die Ansteuersignale für die Dosieranlage längere Zeit ausbleiben, so daß bei Inbetriebnahme mit einer Anzeige auf die Notwendigkeit zu einer Sanierung hingewiesen wird.

Bei der Beschreibung der Figur 1 ist bereits der Konzentrationsmelder 18 erwähnt. Dieser Konzentrationsmelder taucht mit seinen Elektroden 19 in den Behälter 10 ein und mißt nicht nur die Konzentration des Desinfektionsmittels, sondern erkennt und meldet auch, wann Desinfektionsmittel nachgefüllt werden muß. Ein entsprechendes Auswertesignal wird ebenfalls an den MP 20 gegeben. Dieser entscheidet dann aufgrund der Information über den Konzentrationsgehalt des Desinfektionsmittels, ob eine Dauerdosierung oder eine Langzeitdesinfektion notwendig ist und bestimmt so dann Dosierdauer und Einwirkzeit des Desinfektionsmittels. Denkbar ist es auch, den Konzentrationsmelder 18 über den MP 20 mit der Desinfektionsanzeigeleuchte 23 zu koppeln, und zwar derart, daß, wenn keine Desinfektionsflüssigkeit im Behälter ist, die Lampe aufleuchtet.

Das Durchspülen der Leitungsabschnitte mittels Frischwasser erfolgt nach einer Standzeit von etwa 12 bis 24 Stunden, die ebenfalls im Timer 21 vorgewählt und gespeichert werden kann.

Mit 32 ist schließlich noch eine automatische Keimzähleinrichtung bezeichnet, welche der Ablaufsteuereinrichtung 20 die Anzahl der vorhandenen Keime im Wassernetz meldet. Die Ablaufsteuereinrichtung 20 kann so selbsttätig entscheiden, ob eine Langzeitdesinfektion oder eine Dauerdosierung notwendig ist. Bei dieser Variante kann auf den zuvor genannten Desinfektionswahlschalter 30 verzichtet werden.

## Patentansprüche

1. Verfahren zur Desinfektion von Wasserwegen in einem zahnmedizinischen Gerät, welches an ein stationäres Wasserleitungsnetz angeschlossen ist und welchem geräteeingangsseitig über eine Zufuhrleitung (4) unter Zwischenschaltung eines Absperrventils (5) aus diesem Wasserleitungsnetz Wasser zugeführt wird, welches dann in diversen Leitungen (4a bis 4d) zu Verbrauchern (1 bis 3) geleitet wird, denen weitere Absperrmittel (6 bis 8) zugeordnet sind, unter Verwendung einer Steuereinrichtung mit der das Einbringeneines Desinfektionsmittels in die Wasserwege bewirkt werden kann, dadurch gekennzeichnet, daß die Steuereinrichtung als Ablaufsteuereinrichtung (20) ausgebildet ist, mittels der folgende Verfahrensschritte selbsttätig nacheinander angesteuert werden:
a) die Wasserzufuhr wird geräteeingangsseitig (5) abgesperrt,
b) die den einzelnen Verbrauchern (1 bis 3) zugeordneten Absperrmittel (6 bis 8) werden sämtlich geöffnet,
c) sämtliche Wasserwege (4a bis 4d) werden bei geöffneten Absperrmitteln mit einem, vorzugsweise unter Druck eingebrachten. Desinfektionsmittel vollständig gefüllt,
d) nach Ablauf einer Einwirkzeit für das Desinfektionsmittel wird die Wasserzufuhr wieder eingeschaltet und das Desinfektionsmitte aus den Wasserwegen verdrängt.

2. Verfahren nach Anspruch 1, dadurch gekennzeichnet, daß die Ablaufsteuereinrichtung (20) die Absperrmittel (6 bis 8) nacheinander öffnet.

3. Verfahren nach Anspruch 1 oder 2 dadurch gekennzeichnet, daß die Ablaufsteuereinrichtung (20) durch einen manuell betätigbaren Schalter oder eine Taste (26) aktivierbar ist.

4. Verfahren nach Anspruch 1 oder 2, dadurch gekennzeichnet, daß die Ablaufsteuereinrichtung (20) selbsttätig eingeschaltet wird, indem die Ein- und Abschaltzeiten für eine Desinfektion durch einen mit der Ablaufsteuereinrichtung verbundenen Timer (21) vorgegeben werden.

5. Verfahren nach einem der Ansprüche 1 bis 4, dadurch gekennzeichnet, daß der Beginn einer notwendigen Desinfektion mittels eines akustischen und/oder optischen Anzeigeelementes (23) angezeigt wird.

6. Verfahren nach einem der Ansprüche 1 bis 5, dadurch gekennzeichnet, daß ein Zeitspeicherglied (22) vorhanden ist, das die Ablaufsteuereinrichtung (20) und/oder das Anzeigeelement (23) aktiviert, wenn eine vorbestimmte Pausenzeit überschritten wird.

7. Verfahren nach einem der Ansprüche 1 bis 6, dadurch gekennzeichnet, daß der Beginn einer notwendigen Desinfektion durch eine die Keimzahl in den Wasserwegen (4a bis 4d) erfassende Keimzähleinrichtung (32) festgelegt wird.

8. Verfahren nach einem der Ansprüche 1 bis 7, dadurch gekennzeichnet, daß für eine Dauerdesinfektion eine Dosiereinrichtung (11) vorhanden ist, welche Desinfektionsmittel in einer bestimmten Menge dem Wasser zudosiert.

9. Verfahren nach Anspruch 8, dadurch gekennzeichnet, daß die Zudosiereinrichtung (11) mittels der Ablaufsteuereinrichtung (20) ein- und ausgeschaltet wird.

10. Verfahren mit aus Ablagen entnehmbaren Handstücken als Verbraucher nach einem der Ansprüche 1 bis 9, dadurch gekennzeichnet, daß das Ein und Abschalten der Ablaufsteuereinrichtung (20) und/oder der Zudosiereinrichtung (11) durch Entnahmesignalgeber (24) der betreffenden Handstücke (1, 2... n) erfolgt.

11. Verfahren nach Anspruch 10, dadurch gekennzeichnet, daß die Entnahmesignalgeber (24) über eine Logikverknüpfung (25) mit der Ablaufsteuereinrichtung (20) verbunden sind, derart, daß eine Aktivierung der Ablauf steuereinrichtung (20) erst dann erfolgt, wenn sämtliche Handstücke (1, 2... n) aus ihren Ablagen entnommen sind.

12. Verfahren nach einem der Anspruche 1 bis 11, dadurch gekennzeichnet, 3 daß der Verfahrensablauf
a) einer Langzeitdesinfektion nach Anspruch 1,
b) einer Dauerdesinfektion nach Anspruch 8 und
c) einer Dauerdesinfektion mit Langzeitdesinfektionsmeldung nach Anspruch 6 mittels eines Wahlschalters (30) umstellbar ist.

13. Einrichtung zur Desinfektion von Wasserwegen in einem zahnmedizinischen Gerät, welches an ein stationäres Wasserleitungsnetz angeschlossen ist und welchem geräteeingangsseitig über eine Zufuhrleitung (4) unter Zwischenschaltung eines Absperrventils (5) aus diesem Wasserleitungsnetz Wasser zugeführt wird, welches dann in diversen Leitungen (4a bis 4d) zu Verbrauchern (1 bis 3) geleitet wird, denen weitere Absperrmittel (6 bis 8) zugeordnet sind, mit einer Steuereinrichtung mit der das Einbringeneines Desinfektionsmittels in die Wasserwege bewirkt werden kann, dadurch gekennzeichnet, daß die Steuereinrichtung als Ablaufsteuereinrichtung (20) ausgebildet ist, mittels der folgende Verfahrensschritte selbsttätig nacheinander angesteuert werden:
a) die Wasserzufuhr wird geräteeingangsseitig (5) abgesperrt,
b) die den einzelnen Verbrauchern (1 bis 3) zugeordneten Absperrmittel (6 bis 8) werden sämtlich geöffnet,
c) sämtliche Wasserwege (4a bis 4d) werden bei geöffneten Absperrmitteln mit einem, vorzugsweise unter Druck eingebrachten, Desinfektionsmittel vollständig gefüllt,
d) nach Ablauf einer Einwirkzeit für das Desinfektionsmittel wird die Wasserzufuhr wieder eingeschaltet und das Desinfektionsmittel aus den Wasserwegen verdrängt.

14. Einrichtung nach Anspruch 13 dadurch gekennzeichnet, daß die Ablaufsteuereinrichtung (20) durch einen manuell betätigbaren Schalter oder eine Taste (26) aktivierbar ist.

15. Einrichtung nach Anspruch 13 oder 14 dadurch gekennzeichnet, daß die Ablaufsteuereinrichtung (20) selbsttätig eingeschaltet wird, indem die Ein- und Abschaltzeiten für eine Desinfektion durch einen mit der Ablaufsteuereinrichtung verbundenen Timer (21) vorgegeben werden.

16. Einrichtung nach einem der Ansprüche 13 bis 15, dadurch gekennzeichnet, daß der Beginn einer notwendigen Desinfektion mittels eines akustischen und/oder optischen Anzeigeelementes (23) angezeigt wird.

17. Einrichtung nach einem der Ansprüche 13 bis 16, dadurch gekennzeichnet, daß ein Zeitspeicherglied (22) vorhanden ist, das die Ablaufsteuereinrichtung (20) und/oder das Anzeigeelement (23) aktiviert, wenn eine vorbestimmte Pausenzeit überschritten wird.

18. Einrichtung nach einem der Ansprüche 13 bis 17, dadurch gekennzeichnet, daß der Beginn einer notwendigen Desinfektion durch eine die Keimzahl in den Wasserwegen (4a bis 4d) erfassende Keimzähleinrichtung (32) festgelegt wird.

19. Einrichtung nach einem der Ansprüche 13 bis 18, dadurch gekennzeichnet, daß für eine Dauerdesinfektion eine Dosiereinrichtung (11) vorhanden ist, welche Desinfektionsmittel in einer bestimmten Menge dem Wasser zudosiert.

20. Einrichtung nach Anspruch 19, dadurch gekennzeichnet, daß die Zudosiereinrichtung (11) mittels der Ablaufsteuereinrichtung (20) ein- und ausgeschaltet wird.

21. Einrichtung mit aus Ablagen entnehmbaren Handstücken als Verbraucher, nach einem der Ansprüche 13 bis 20, dadurch gekennzeichnet, daß das Ein und Abschalten der Ablaufsteuereinrichtung (20) und/oder der Zudosiereinrichtung (11) durch Entnahmesignalgeber (24) der betreffenden Handstücke (1, 2... n) erfolgt.

22. Einrichtung nach Anspruch 21, dadurch gekennzeichnet, daß die Entnahmesignalgeber (24) über eine Logikverknüpfung (25) mit der Ablaufsteuereinrichtung (20) verbunden sind, derart, daß eine Aktivierung der Ablauf steuereinrichtung (20) erst dann erfolgt, wenn sämtliche Handstücke (1, 2... n) aus ihren Ablagen entnommen sind.

23. Einrichtung nach einem der Ansprüche 13 bis 22, dadurch gekennzeichnet, daß in einem das Desinfektionsmittel aufnehmenden Behälter (10) Füllstandsmelder (19) vorhanden sind, die bei Unterschreiten eines minimalen Füllstandes eine Anzeige liefern.

24. Einrichtung nach Anspruch 23, dadurch gekennzeichnet, daß die Füllstandsmelder (19) gleichsam als Konzentrationsmelder für das verwendete Desinfektionsmittel ausgebildet sind.

25. Einrichtung nach einem der Ansprüche 13 bis 24, dadurch gekennzeichnet, daß als Ablaufsteuereinrichtung ein Mikroprozessor (20) vorgesehen ist.

26. Einrichtung nach einem der Ansprüche 13 bis 25, dadurch gekennzeichnet, daß als Desinfektionsmittel für eine Langzeitdesinfektion Chlorhexidindigluconat in einer Konzentration zwischen 0,05 und 0,2 verwendet wird.

27. Einrichtung nach einem der Ansprüche 13 bis 26, dadurch gekennzeichnet, daß für eine Dauerdesinfektion Chlorhexidin in einer Menge zwischen 5 ppm und 1 ppm dem Wasser zudosiert wird.

28. Einrichtung nach Anspruch 26, dadurch gekennzeichnet, daß nach der Desinfektion mit Chlorhexidindigluconat ein Silberpräparat in einer Menge von 0,05 bis 0,1 mg/l Wasser in die Wasserwege eingebracht wird.

## Claims

1. Process for disinfecting water ducts in a dental appliance which is connected to a fixed water supply system and to which water is supplied from this water supply system on the inlet side of the appliance by way of a supply line (4) with the interposition of a shut-off valve (5) and is then conveyed in various supply lines (4a to 4d) to consumers (1 to 3) to which there are assigned further shut-off means (6 to 8), with the use of a control device by means of which a disinfectant can be introduced into the water ducts, characterised in that the control device is formed as a sequence control device (20) by which the following process steps are initiated automatically one after another:
a) the water supply is shut off on the inlet side of the appliance,
b) the shut-off means (6 to 8) assigned to the individual consumers (1 to 3) are all opened
c) when the shut-off means (6 to 8) are open, all of the water ducts (4a to 4d) are filled completely with a disinfectant which is preferably introduced under pressure,
d) after the expiration of a period for the disinfectant to act, the water supply is reconnected and the disinfectant is driven out of the water ducts.

2. Process according to claim 1, characterised in that the sequence control (20) opens the shut-off means (6 to 8) one after another.

3. Process according to claim 1 or 2, characterised in that the sequence control device (20) can be activated by a manually operated switch or by a push-button (26).

4. Process according to claim 1 or 2, characterised in that the sequence control device (20) is connected automatically, the switched-on and switched-off times for disinfection being preselected by a timer (21) connected to the sequence control device (20).

5. Process according to one of claims 1 to 4, characterised in that the start of necessary disinfection is indicated by means of an acoustic and/or optical indicating element (23).

6. Process according to one of claims 1 to 5, characterised in that there is a time register element (22) which activates the sequence control device (20) and/or the indicating element (23) when a predetermined time interval has been exceeded.

7. Process according to one of claims 1 to 6, characterised in that the start of necessary disinfection is determined by a germ counting device (32) which detects the number of germs in the water ducts (4a to 4d).

8. Process according to one of claims 1 to 7, characterised in that, for continuous disinfection, there is a dosing device (11) which adds a predetermined dosed amount of disinfectant to the water.

9. Process according to claim 8, characterised in that the dosing device (11) is switched on and off by the sequence control device (20).

10. Process having handpieces as consumers which can be teen out of receptacles, according to one of claims 1 to 9, characterised in that switching on and off of the sequence control device (20) and/or the dosing device (11) is brought about by means of sampling signal emitters (24) of the corresponding handpieces (1, 2 ... n).

11. Process according to claim 10, characterised in that the sampling signal emitters (24) are connected to the sequence control device (20) by way of a logic circuit (25) in such a way that activation of the sequence control device (20) only takes place when all of the handpieces (1, 2 ... n) have been taken out of their receptacles.

12. Process according to any one of claims 1 to 11, characterised in that the process sequence comprising:
a) long-duration disinfection according to claim 1,
b) continuous disinfection according to claim 8, and
c) continuous disinfection with long-duration disinfection indication according to claim 6,
can be changed by means of a selector switch (30).

13. Device for disinfecting water ducts in a dental appliance which is connected to a fixed water supply system and to which water is supplied from this water supply system on the inlet side of the appliance by way of a supply line (4) with the interposition of a shut-off valve (5) and is then conveyed in various supply lines (4a to 4d) to consumers (1 to 3) to which there are assigned further shut-off means (6 to 8), having a control device by means of which a disinfectant can be introduced into the water ducts, characterised in that the control device is formed as a sequence control device (20) by which the following process steps are initiated automatically one after another:
a) the water supply is shut off on the inlet side (5) of the appliance,
b) the shut-off means (6 to 8) assigned to the individual consumers (1 to 3) are opened together,
c) when the shut-off means (6 to 8) are open, all of the water ducts (4a to 4d) are filled completely with a disinfectant which is preferably introduced under pressure,
d) after the expiration of a period for the disinfectant to act, the water supply is reconnected and the disinfectant is driven out of the water ducts.

14. Device according to claim 13, characterised in that the sequence control device (20) can be activated by a manually operated switch or by a push-button (26).

15. Device according to claim 13 or 14, characterised in that the sequence control device (20) is connected automatically, the switched-on and switched-off times for disinfection being preselected by a timer (21) connected to the sequence control device.

16. Device according to one of claims 13 to 15, characterised in that the start of necessary disinfection is indicated by means of an acoustic and/or optical indicating element (23).

17. Device according to one of claims 13 to 16, characterised in that there is a time register element (22) which activates the sequence control device (20) and/or the indicating element (23) when a predetermined time interval has been exceeded.

18. Device according to one of claims 13 to 17, characterised in that the start of necessary disinfection is determined by a germ counting device (32) which detects the number of germs in the water ducts (4a to 4d).

19. Device according to one of claims 13 to 18, characterised in that, for continuous disinfection, there is a dosing device (11) which adds a predetermined dosed amount of disinfectant to the water.

20. Device according to claim 19, characterised in that the dosing device (11) is switched on and off by the sequence control device (20).

21. Device having handpieces as consumers which can be taken out of receptacles, according to one of claims 13 to 20, characterised in that switching on and off of the sequence control device (20) and/or the dosing device (11) is brought about by means of sampling signal emitters (24) of the corresponding handpieces (1, 2 ... n).

22. Device according to claim 21, characterised in that the sampling signal emitters (24) are connected to the sequence control device (20) by way of a logic circuit (25) in such a way that activation of the sequence control device (20) only takes place when all of the handpieces (1, 2 ... n) have been taken out of their receptacles.

23. Device according to one of claims 13 to 22, characterised in that, in a container (10) holding the disinfectant, there are liquid level indicators (19) which supply an indication when the liquid falls below a minimum level.

24. Device according to claim 23, characterised in that the liquid level indicators (19) are formed as concentration indicators for the disinfectant which is used.

25. Device according to one of claims 13 to 24, characterised in that a microprocessor (20) is provided as the sequence control device.

26. Device according to one of claims 13 to 25, characterised in that a concentration of between 0.05 and 0.2 of chlorhexidine digluconate is used as disinfectant for long-duration disinfection.

27. Device according to one of claims 13 to 26, characterised in that, for continuous disinfection, a dosed amount of between 5 ppm and 1 ppm of chlorhexidine is added to the water.

28. Device according to claim 26, characterised in that, after disinfection with chlorhexidine digluconate, an amount of between 0.05 and 0.1 mg of silver preparation per litre of water is introduced into the water ducts.

## Revendications

1. Procédé de désinfection de voies d'eau dans un appareil de la médecine dentaire qui est raccordé à un réseau de distribution d'eau et auquel de l'eau est amenée du côté de l'entrée de l'appareil par un conduit d'amenée (4) avec interposition d'une vanne d'arrêt (5), l'eau étant envoyée ensuite par divers conduits (4a à 4d) à des appareils utilisateurs (1 à 3) auxquels sont associés d'autres moyens d'arrêt (6 à 8) en utilisant un dispositif de commande par lequel on peut faire en sorte qu'un agent désinfectant soit introduit dans la voie d'eau, caractérisé en ce que le dispositif de commande est conçu en tant que dispositif de commande du déroulement (20), au moyen duquel les stades suivants du procédé sont commandés automatiquement l'un après l'autre :
a) l'amenée d'eau est fermée du côté de l'entrée de l'appareil (5),
b) les moyens d'arrêt (6 à 8) associés aux divers appareils utilisateurs (1 à 3) sont tous ouverts,
c) toutes les voies d'eau (4a à 4b) sont, alors que les moyens d'arrêt sont ouverts, entièrement emplies d'un agent désinfectant introduit, de préférence, sous pression,
d) après qu'une durée d'action de l'agent désinfectant se soit écoulée, l'amenée d'eau est branchée à nouveau et l'agent désinfectant est refoulé des voies d'eau.

2. Procédé suivant la revendication 1, caractérisé en ce que le dispositif de commande de déroulement (20) ouvre les moyens d'arrêt (6 à 8) l'un après l'autre.

3. Procédé suivant la revendication 1 ou 2, caractérisé en ce que le dispositif de commande de déroulement (20) est mis en action par un interrupteur manoeuvrable à la main ou par une touche (26).

4. Procédé suivant la revendication 1 ou 2, caractérisé en ce que le dispositif de commande de déroulement (20) est mis en route automatiquement, en prescrivant les durées de mise en route et d'arrêt pour une désinfection, par une minuterie (21) reliée au dispositif de commande de déroulement.

5. Procédé suivant l'une des revendications 1 à 4, caractérisé en ce que le début de la necessité d'une désinfection est indiqué au moyen d'un élément d'indication (23) acoustique et/ou optique.

6. Procédé suivant l'une des revendications 1 à 5, caractérisé par un élément de mémorisation du temps (22) qui met en action le dispositif de commande du déroulement (20) et/ou l'élément indicateur (23), quand un temps de repos prescrit est dépassé.

7. Procédé suivant l'une des revendications 1 à 6, caractérisé en ce que le début de la nécessité d'une désinfection est fixé par un dispositif de dénombrement des germes (32) détectant le nombre de germes dans les voies d'eau (4a à 4d).

8. Procédé suivant l'une des revendications 1 à 7, caractérisé en ce que, pour une désinfection permanente, il est prévu un dispositif de dosage (11) qui ajoute, de manière dosée, de l'agent désinfectant à l'eau en une quantité déterminée.

9. Procédé suivant la revendication 8, caractérisé en ce que le dispositif (11) d'addition en quantité dosée est mise en service et hors service au moyen du dispositif de commande de déroulement (20).

10. Procédé ayant comme appareil utilisateur des pièces à main pouvant être enlevées de dispositifs de rangement, suivant l'une des revendications 1 à 9, caractérisé en ce que la mise en circuit et hors circuit du dispositif de commande de déroulement (20) et/ou du dispositif d'addition en quantités dosées (11) s'effectue par des générateurs de signaux d'enlèvement (24) des pièces à main concernées (1, 2 ... n).

11. Procédé suivant la revendication 10, caractérisé en ce que les générateurs de signaux d'enlèvement (24) sont reliés par une unité logique (25) au dispositif de commande de déroulement (20), de façon à n'effectuer une mise en action du dispositif de commande de déroulement (20) que lorsque toutes les pièces à main (1, 2 ... n) ont été retirées de leur dispositif de rangement.

12. Procédé suivant l'une des revendications 1 à 11, caractérisé en ce que le déroulement du procédé peut être branché au moyen d'un commutateur de sélection (30)
a) sur une désinfection de longue durée suivant la revendication 1,
b) sur une désinfection permanente, suivant la revendication 8, et
c) sur une désinfection permanente, avec indication de la désinfection de longue durée, suivant la revendication 6.

13. Dispositif de désinfection de voies d'eau dans un appareil de la médecine dentaire qui est raccordé à un réseau de distribution d'eau et auquel de l'eau est amenée du côté de l'entrée de l'appareil par un conduit d'amenée (4) avec interposition d'une vanne d'arrêt (5), l'eau étant envoyée ensuite par divers conduits (4a à 4d) à des appareils utilisateurs (1 à 3) auxquels sont associés d'autres moyens d'arrêt (6 à 8) en utilisant un dispositif de commande par lequel on peut faire en sorte qu'un agent désinfectant soit introduit dans la voie d'eau, caractérisé en ce que le dispositif de commande est conçu en tant que dispositif de commande du déroulement (20), au moyen duquel les stades suivants du procédé sont commandés automatiquement l'un après l'autre :
a) l'amenée d'eau est fermée du côté de l'entrée de l'appareil (5),
b) les moyens d'arrêt (6 à 8) associés aux divers appareils utilisateurs (1 à 3) sont tous ouverts,
c) toutes les voies d'eau (4a à 4b) sont, alors que les moyens d'arrêt sont ouverts, entièrement emplies d'un agent désinfectant introduit, de préférence, sous pression,
d) après qu'une durée d'action de l'agent désinfectant se soit écoulée, l'amenée d'eau est branchée à nouveau et l'agent désinfectant est refoulé des voies d'eau.

14. Dispositif suivant la revendication 13, caractérisé en ce que le dispositif de commande de déroulement (20) est mis en action par un interrupteur manoeuvrable à la main ou par une touche (26).

15. Dispositif suivant la revendication 13 ou 14, caractérisé en ce que le dispositif de commande de déroulement (20) est mis en route automatiquement, en prescrivant les durées de mise en route et d'arrêt pour une désinfection, par une minuterie (21) reliée au dispositif de commande de déroulement.

16. Dispositif suivant les revendications 13 à 15, caractérisé en ce que le début de la necessité d'une désinfection est indiqué au moyen d'un élément d'indication (23) acoustique et/ou optique.

17. Dispositif suivant l'une des revendications 13 à 16, caractérisé par un élément de mémorisation du temps (22) qui met en action le dispositif de commande du déroulement (20) et/ou l'élément indicateur (23), quand un temps de repos prescrit est dépassé.

18. Dispositif suivant l'une des revendications 13 à 17, caractérisé en ce que le début de la nécessité d'une désinfection est fixé par un dispositif de dénombrement des germes (32) détectant le nombre de germes dans les voies d'eau (4a à 4d).

19. Dispositif suivant l'une des revendications 13 à 18, caractérisé en ce que, pour une désinfection permanente, il est prévu un dispositif de dosage (11) qui ajoute, de manière dosée, de l'agent désinfectant à l'eau en une quantité déterminée.

20. Dispositif suivant la revendication 19, caractérisé en ce que le dispositif (11) d'addition en quantité dosée est mise en service et hors service au moyen du dispositif de commande de déroulement (20).

21. Dispositif ayant comme appareil utilisateur des pièces à main pouvant être enlevées de dispositifs de rangement suivant l'une des revendications 13 à 20, caractérisé en ce que la mise en circuit et hors circuit du dispositif de commande de déroulement (20) et/ou du dispositif d'addition en quantités dosées (11) s'effectue par des générateurs de signaux d'enlèvement (24) des pièces à main concernées (1, 2 ... n).

22. Dispositif suivant la revendication 21, caractérisé en ce que les générateurs de signaux d'enlèvement (24) sont reliés par une unité logique (25) au dispositif de commande de déroulement (20), de façon à n'effectuer une mise en action du dispositif de commande de déroulement (20) que lorsque toutes les pièces à main (1, 2 ... n) ont été retirées de leur dispositif de rangement.

23. Dispositif suivant l'une des revendications 13 à 22, caractérisé en ce que, dans un réservoir (10) de réception de l'agent désinfectant, sont prévus des indicateurs de niveau (19), qui fournissent une indication lorsque le niveau passe en-dessous d'un niveau minimum.

24. Dispositif suivant la revendication 23, caractérisé en ce que les indicateurs de niveau (19) sont constitués, en quelque sorte en indicateurs de concentration de l'agent désinfectant utilisé;

25. Procédé suivant l'une des revendications 13 à 24, caractérisé en ce qu'il est prévu un microprocesseur (20) comme dispositif de commande de déroulement.

26. Dispositif suivant l'une des revendications 13 à 25, caractérisé en ce que du gluconate de chlorohexidine en une concentration comprise entre 0,05 et 0,2 % est utilisé comme agent désinfectant pour une désinfection de longue durée.

27. Dispositif suivant l'une des revendications 13 à 26, caractérisé en ce que de la chlorohexidine est ajoutée à l'eau en une quantité dosée comprise entre 5 ppm et 1 ppm pour une désinfection permanente.

28. Dispositif suivant la revendication 26, caractérisé en ce qu'après la désinfection par du digluconate de chlorohexidine, une préparation à l'argent en une quantité de 0,05 à 0,1 mg/l d'eau est introduite dans les voies d'eau.
